# EUROPEAN PATENT APPLICATION

(11) **EP 2 742 867 A1**
(43) Date of publication of application: **18.06.2014**
(21) Application number: 12821433.5
(22) Date of filing: 30.05.2012
(51) Int. Cl.: A61B 8/00

(54) **ULTRASOUND DIAGNOSTIC DEVICE AND ULTRASOUND IMAGE DISPLAY METHOD**

(30) Priority: 11.08.2011 JP 2011176152
(71) Applicant: Hitachi Medical Corporation, Tokyo 101-0021 (JP)
(72) Inventor: KONDOU,Masanao, Tokyo 101-0021 (JP)
(74) Representative: MERH-IP Matias Erny Reichl Hoffmann
(86) International application number: PCT/JP2012/063847
(87) International publication number: WO 2013/021711

(57) **Abstract**

An object of the present invention is to provide an ultrasound diagnostic device being able to display an ultrasound image which is not limited to a region targeted for ultrasound emission. The ultrasound diagnostic device 10 is provided with an ultrasound probe 2 having an ultrasound emitting surface for emitting an ultrasound wave and receiving a reflected wave of the ultrasound wave, an ultrasound image generating means 6 for generating an ultrasound image by using an reflected echo signal based on the reflected wave, an ultrasound volume data generating means 9 for accumulating the ultrasound images to generate three-dimensional ultrasound volume data of a test object, a reference image generating means 12 for generating multiple reference images by using three-dimensional volume data and the ultrasound volume data, and a display means 15 for displaying the ultrasound image and the reference images.

## Description

### Technical Field

The present invention relates to an ultrasound diagnostic device and an ultrasound image display method, and more particularly, it relates to a technique for displaying an ultrasound image.

### Background Art

In diagnosis using an ultrasound diagnostic device, an operator such as a doctor scans a diagnosis portion by an ultrasound probe, and this provides an advantage that it is possible to easily obtain a tomographic image of the diagnosis portion in real time. On the other hand, an ultrasound tomographic image (hereinafter, an ultrasound image) is less easily viewable as morphological information of a whole body of test object, than a tomographic image obtained by a magnetic resonance imaging device (hereinafter, referred to as "MRI device") or an X-ray computed tomography scanner (hereinafter, referred to as "X-ray CT scanner").

Therefore, it is requested that not only an ultrasound image, but also an image taken by the MRI device (hereinafter, referred to as "MRI image"), an image taken by the X-ray CT scanner (hereinafter, referred to as "CT image"), and an ultrasound image made up of multiple images of an affected area previously taken by the ultrasound diagnostic device (hereinafter, referred to as "ultrasound volume data"), and the like, are displayed together, and a comprehensive diagnosis is conducted while comparing those images with one another.

By way of example, the Patent Document 1 discloses an ultrasound diagnostic device and an ultrasound image display method for detecting a position and a posture of an ultrasound endoscope based on a position detection sensor being mounted on the ultrasound endoscope, thereby reconstructing an image of the same cross section as that of the ultrasound image, based on volume data of an MRI image and a CT image being taken in advance, synchronizing the reconstructed image with the ultrasound image, and displaying those images on a monitor.

### Prior Art Document

### Patent Document

Patent Document 1: Japanese Unexamined Patent Application Publication No. 2009-095371

### Disclosure of the Invention

### Problem to be solved by the Invention

In the Patent Document 1, an ultrasound image is displayed simultaneously with an MRI image and a CT image of a cross section that is identical to the cross section of the ultrasound image, and this enhances the quality of morphological information. However, since the cross section of the MRI image or the CT image is identical to the cross section of the ultrasound image, there is no degree of freedom for the user.

The present invention has been made in view of the above problem, and an object of the present invention is to provide an ultrasound diagnostic device and an ultrasound image display method being able to display a reference image that is reconstructed from volume data, and an area of the reference image is not limited to a region targeted for ultrasound emission.

### Means to solve the Problem

In order to solve the aforementioned problem, the ultrasound diagnostic device relating to the present invention is provided with an ultrasound probe having an ultrasound emitting surface for emitting an ultrasound wave and receiving a reflected wave of the ultrasound wave, an ultrasound image generating means for generating an ultrasound image by using an reflected echo signal based on the reflected wave, a reference image generating means for generating reference images of multiple arbitrary cross sections by using three-dimensional volume data of a test object, and a display means for displaying the ultrasound image and the reference images. In addition, the ultrasound image display method is provided with a step of emitting an ultrasound wave and generating an ultrasound image by using a reflected echo signal based on a reflected wave of the ultrasound wave, a step of generating reference images of multiple arbitrary cross sections by using three-dimensional volume data of a test object, and a step of displaying the ultrasound image and the reference images.

### Effect of the Invention

According to the present invention, it is possible to provide an ultrasound diagnostic device and an ultrasound image display method being able to display an ultrasound image that is not limited to a region targeted for ultrasound emission.

### Brief Description of Drawings

FIG. 1 is a diagram schematically illustrating an overall configuration of the ultrasound diagnostic device;
FIG. 2 illustrates a schematic configuration of the ultrasound probe 2;
FIG. 3 illustrates a positional relationship between the ultrasound emitting surface and the ultrasound image, and a positional relationship among the first reference image, the second reference image, and the third reference image; FIG. 3(a) illustrates a positional relationship between the ultrasound emitting surface and the ultrasound image emitted therefrom, and FIG. 3(b) illustrates a positional relationship among the cross sections constituting the respective reference images;
FIG. 4 illustrates a screen display example of the first embodiment;
FIG. 5 illustrates a process for moving the second reference image, in the upward and downward directions, in the right and left directions, and in an oblique direction;
FIG. 6 illustrates movement and rotation of the second reference image; FIG. 6 (a) illustrates the state where the second reference image is moved in the front direction and the rear direction, and FIG. 6(b) illustrates the state where the second reference image is rotated, assuming the direction for inserting the ultrasound prove as the rotation axis;
FIG. 7 illustrates rotation of the second reference image; FIG. 7 (a) illustrates the state where the second reference image is rotated assuming the depth direction as the rotation axis, and FIG. 7(b) illustrates the state where the second reference image is rotated assuming the front-and-rear direction as the rotation axis;
FIG. 8 illustrates a method for moving the reference image according to a guide image;
FIG. 9 is a diagram illustrating a screen display example being displayed in the second embodiment;
FIG. 10 illustrates a process for generating a reference image 60 from the second reference image 36,
FIG. 10(a) shows the tip of the ultrasound probe A, and FIG. 10(b) illustrates a process for generating the reference image 60 from the second reference image 36; and
FIG. 11 illustrates a process for generating the reference image 60 from the third reference image 38.

### Modes for carrying out the Invention

Hereinafter, preferred embodiments of the present invention will be explained with reference to the drawings. It is to be noted that in the following description, a constituent having the same function will be labeled the same, and tedious explanation will not be made.

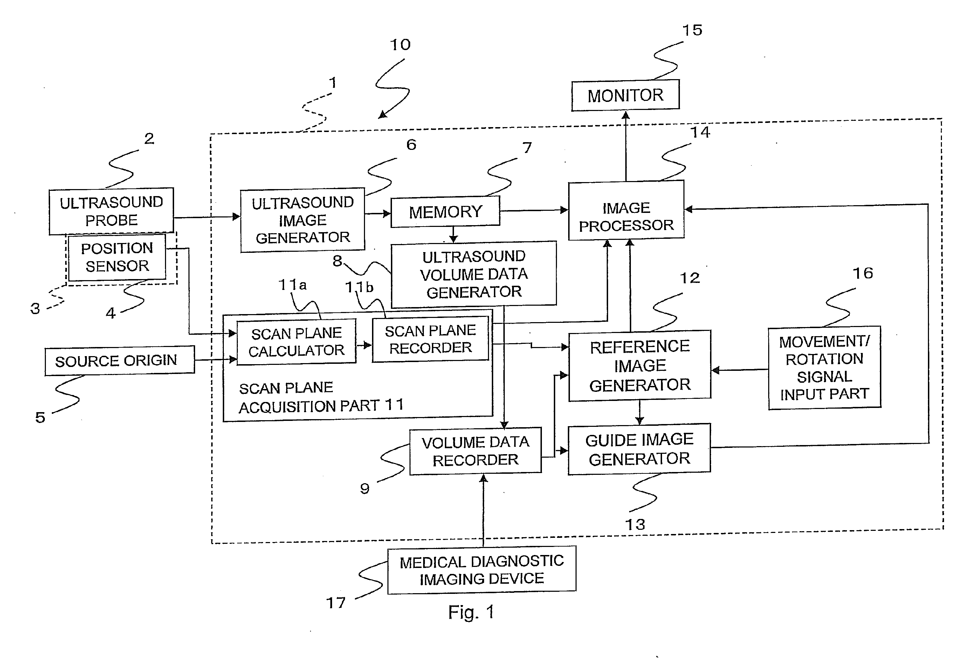

FIG. 1 is a diagram illustrating an overall schematic configuration of the ultrasound diagnostic device relating to the embodiments of the present invention. The ultrasound diagnostic device 10 incorporates as primary parts, an ultrasound diagnostic device main unit 1, an ultrasound probe 2, a position sensor 4, a source origin 5, and a monitor 15. The ultrasound diagnostic device main unit 1 is roughly divided into a system for generating an ultrasound image, and a system for generating a reference image used for reference upon performing a diagnosis on the ultrasound image.

The system for generating the ultrasound image includes, an ultrasound image generator 6 for generating an ultrasound image based on a reflected echo signal from the ultrasound probe 2, a memory 7 for temporarily storing multiple ultrasound images, and an ultrasound volume data generator 8 for generating three-dimensional ultrasound volume data based on the multiple ultrasound images. The system for generating the reference image is provided with a volume data recorder 9 for storing the three-dimensional volume data taken by a medical diagnostic imaging device 17 such as an MRI device, a CT scanner, and other ultrasound diagnostic device, for instance, a scan plane acquisition part 11 for detecting a position and a posture of the ultrasound probe 2, based on signals of the position sensor 4 and the source origin 5, a reference image generator 12 for generating a reference image based on the three-dimensional volume data and the ultrasound volume data stored in the volume data recorder 9, the reference image being used for reference upon performing a diagnosis on the ultrasound image, a guide image generator 13 for generating a guide image indicating a cross section position of the reference image, and a movement/rotation signal input part 16 for accepting inputs of a movement/rotation type, a moving amount/rotating amount, and a moving direction/rotating direction of the reference image. In addition, the ultrasound diagnostic device main unit 1 includes an image processor 14 for performing a display process, establishing an association between the cross section positions of the ultrasound image and the reference image.

Though not illustrated, it is to be noted that the ultrasound diagnostic device main unit 1 is equipped with an interface for inputting the three-dimensional volume data of the test object imaged by the medical diagnostic imaging device 17, the ultrasound volume data imaged by other ultrasound diagnostic device, and the like. Then, the ultrasound diagnostic device main unit 1 is directly connected to the medical diagnostic imaging device 17 via the interface, receives the three-dimensional volume data, and stores the volume data in the volume data recorder 9. It is further possible to store the three-dimensional volume data in the volume data recorder 9 within the ultrasound diagnostic device main unit, via a network or via a portable recording medium, such as a USB memory. Hereinafter, each constitutional element will be explained in detail.

With the aforementioned constitutional elements, the ultrasound diagnostic device main unit 1 is allowed to generate a reference tomographic image having the same cross section, the same magnification, and the same field of view as those of the ultrasound tomographic image obtained by the ultrasound probe 2, thereby generating a composite image. Then, this composite image and the guide image are displayed on the same screen of the monitor 15 of the ultrasound diagnostic device 10. Therefore, the operator is allowed to easily grasp a correspondence relationship between the ultrasound image and the reference image, and by contrasting those images, it is possible to perform a comprehensive diagnosis effectively on the test object.

The ultrasound probe 2 transfers an ultrasound wave from the ultrasound emitting surface, also receives a reflected wave of the ultrasound wave, and outputs a reflected echo signal to the ultrasound image generator 6.

The ultrasound image generator 6 generates an ultrasound image for one frame, at each position of the ultrasound probe 2. Then, along with shifting the position of the ultrasound probe 2, the ultrasound image generator generates ultrasound images respectively associated with multiple frames, at multiple positions. The memory 7 temporarily stores the ultrasound images associated with the multiple frames. The ultrasound volume data generator 8 generates three-dimensional ultrasound volume data that is obtained by accumulating the ultrasound images of the multiple frames along one direction (the moving direction of the ultrasound probe 2), based on the ultrasound images temporarily stored in the memory 7. The volume data recorder 9 records the three-dimensional ultrasound volume data thus generated, and also records the three-dimensional volume data imaged by the medical diagnostic imaging device 17.

The position sensor 4 such as a magnetic sensor for detecting the position and posture of the ultrasound probe 2 is fixed on the ultrasound probe 2 via the position sensor fixing mechanism 3 that is mounted on the ultrasound probe 2. In addition, the source origin 5 for generating source such as magnetic field in the coordinate system including the test object, is arranged on the side of the bed on which the test object is laid, for instance. The position sensor 4 and the source origin 5 are electrically connected to the scan plane acquisition part 11, and signals from the position sensor 4 and the source origin 5 are outputted to the scan plane acquisition part 11.

The scan plane acquisition part 11 is provided with a scan plane calculator 11a and a scan plane recorder 11b; the scan plane calculator 11a acquires positional information such as the position and inclination angle of the ultrasound probe 2 based on the signals outputted from the position sensor and the source origin 5, calculating the three-dimensional position, inclination angle, and the like, of the ultrasound probe 2, so as to obtain the coordinates of the scan plane (the cross section of the ultrasound image) of the ultrasound probe 2, and the scan plane recorder 11b records the coordinates of the scan plane being obtained. In the present embodiment, the detection of position by using the magnetic field is taken as an example for detecting the position of the ultrasound probe 2. However, this is not the only example, and it is possible to employ a different position detecting method being publicly known. The coordinates of the scan plane being obtained are outputted to the reference image generator 12 and the image processor 14.

The reference image generator 12 uses the coordinates obtained by the scan plane acquisition part 11, so as to generate from the volume data recorded in the volume data recorder 9, a reference image having the same cross section as that of the ultrasound image (hereinafter, referred to as "the first reference image"), a reference image that is obtained by rotating the first reference image by 90° or 270°, assuming the depth direction thereof as a rotation axis (hereinafter, referred to as "the second reference image"), a reference image being parallel to the ultrasound emitting surface of the ultrasound probe 2 (hereinafter, referred to as "the third reference image"), and further a reference image of an optional cross section. Each of the reference images being generated is subjected to a processing such as showing a dotted line indicating the position of another reference image in such a manner as superimposed thereon, or hiding the dotted line.

The reference image generator 12 calculates the coordinates of the ultrasound emission area (also referred to as "FOV") within the ultrasound image based on the scan plane coordinates, and performs processing such as reducing brightness of the areas other than the ultrasound emission area, on the first, the second, and the third reference images, or hiding the area other than the ultrasound emission area.

Furthermore, the reference image generator 12 changes the image size and the frame rate of the reference image according to the movement of the ultrasound probe 2, thereby varying the speed for reconstructing the reference image. In other words, if the ultrasound probe 2 moves quickly in the moving direction, a higher priority is placed on the frame rate than the image quality, and the reference image is depicted at high speed. On the other hand, if the ultrasound probe 2 moves slowly, a higher priority is placed on the image quality than the frame rate in reconstructing and depicting the reference image. This allows the ultrasound image to follow the movement of the ultrasound probe 2, and accordingly this enables the reference image to be depicted.

The reference image generator 12 outputs to the image processor 14, the positional information indicating the cross section position of each reference image and the reference image being generated. The positional information indicating the cross section position of each reference image is also outputted to the guide image generator 13.

The guide image generator 13 uses the three-dimensional volume data recorded in the volume data recorder 9 and the cross-section positional information of the reference image obtained from the reference image generator 12, so as to generate a guide image being displayed in such a manner that the cross section of the reference image in semitransparent color is superimposed on the three-dimensional image of the test object. As a method for generating the three-dimensional image of the test object, to be used for the guide image, a well-known method may be applied, for example, volume rendering, surface rendering, and the like. The guide image being generated is outputted to the image processor 14.

The image processor 14 is connected to the scan plane acquisition part 11, the memory 7, the reference image generator 12, and the guide image generator 13. Then, the image processor acquires an ultrasound image from the memory 7, a reference image from the reference image generator 12, and a guide image from the guide image generator 13. Then, the coordinates of the scan plane in the scan plane acquisition part 11 and the cross-section positional information of the reference image are used to perform processing for superimposing/hiding the dotted line indicating the position of the reference image on the ultrasound image. Furthermore, the image processor performs a processing for establishing an association among the positions of the ultrasound image, the reference image, and the guide image, so as to display those images on the monitor 15. By way of example, the ultrasound image and the reference image may be displayed side by side, or the reference image may be rendered semi-transparent and displayed in such a manner as superimposed on the ultrasound image. If it is superimposed thereon, only one image enables easy comparison between the ultrasound image and the reference image. It is further possible that the images in the memory 7, the images generated in the reference image generator 12 and in the guide image generator 13, are combined appropriately and displayed.

In addition, the image processor 14 performs image processing for superimposing the scan plane in semi-transparent color on the guide image. Accordingly, the operator is allowed to grasp the positional relationship three-dimensionally, between the test object and the scan plane of the ultrasound probe 2.

The movement/rotation signal input part 16 is connected to input units including a pointing device such as a mouse and a track ball, and a keyboard, etc. The operator manipulates those input units to enter a selection either movement or rotation, a selection either moving direction or rotating direction, and a moving amount or a rotating angle. Then, the movement/rotation signal input part 16 acquires those inputted values, and outputs them to the reference image generator 12 and the guide image generator 13. The movement/rotation signal input part 16 may also accept an input for selecting an image targeted for moving or rotating.

The reference image generator 12 moves or rotates the reference image according to the inputted value, and the guide image generator 13 moves the cross section position of the reference image on the guide image. Following this movement, the image processor 14 shifts a mark indicating the cross section position of the reference image being superimposed on the ultrasound image. Alternatively, when the cross section position of the reference image that is superimposed on the guide image or the cross section position of the reference image that is superimposed on the ultrasound image is moved and/or rotated by using the operating unit including pointing devices, such as the mouse and the trackball, the movement/rotation signal input part 16 detects the moving amount and the rotating amount. Then, the reference image generator 12 generates a new reference image according to the inputted values being detected, and the reference image on the monitor 15 is also updated and displayed.

Furthermore, the ultrasound diagnostic device 10 is provided with an operation input unit for zooming in and out on an observation site. Upon zooming in and out on the observation site, a display magnification of the observation site is changed in the ultrasound image that is generated by the ultrasound image generator 6. Following this change, the reference image generator 12 changes the display magnification of the reference image (or generates a new reference image) so as to coincide with the new display magnification of the ultrasound image. The ultrasound image or the reference image whose display magnification has been changed is updated and displayed on the monitor 15.

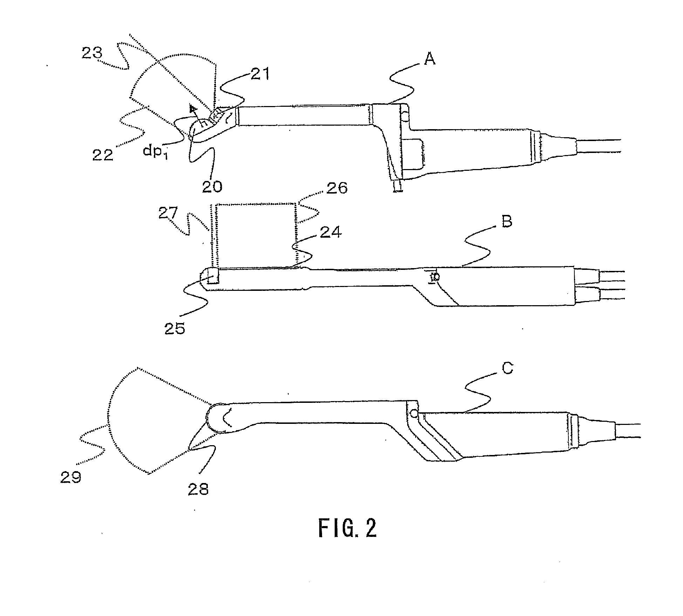

Next, with reference to FIG. 2, the ultrasound probe 2 will be explained. FIG. 2 illustrates a schematic configuration of the ultrasound probe 2.

As the ultrasound probe 2, it is possible to employ a biplane-type ultrasound probe A for acquiring two ultrasound images simultaneously from two ultrasound emitting surfaces, a non-biplane type ultrasound probe B for acquiring one ultrasound image by switching two ultrasound emitting surfaces, and an ultrasound probe C provided with one ultrasound emitting surface.

The ultrasound probe A is inserted into a body cavity of the test object, including a prostatic region, and sends and receives ultrasound waves within the body cavity. The ultrasound probe A is provided with two ultrasound emitting surfaces 20 and 21. The ultrasound emitting surface 20 is mounted on the tip of the ultrasound probe A. Then, the depth direction dp1 of the ultrasound wave emitted from the ultrasound emitting surface 20 is vertical to the ultrasound emitting surface 20. An image taken by the ultrasound wave emitted from the ultrasound emitting surface 20 corresponds to the first cross-section image 22. The ultrasound emitting surface 21 is provided closer to the center of the ultrasound probe A than the ultrasound emitting surface 21. The depth direction of the ultrasound wave emitted from the ultrasound emitting surface 21 is vertical to the ultrasound emitting surface 21. An image taken by the ultrasound wave emitted from the ultrasound emitting surface 21 corresponds to the second cross-section image 23.

The ultrasound probe B is provided with two ultrasound emitting surfaces 24 and 25, and it is a non-biplane type ultrasound probe that acquires an ultrasound image from either one of the ultrasound emitting surfaces, by switching between the ultrasound emitting surfaces 24 and 25. An image obtained from the ultrasound emitting surface 24 corresponds to the third cross-section image 26, and an image obtained from the ultrasound emitting surface 25 corresponds to the fourth cross-section image 27. The depth directions of the ultrasound emitting surfaces 24 and 25 are vertical to the respective ultrasound emitting surfaces.

The ultrasound probe C is an ultrasound probe that is provided with one ultrasound emitting surface 28. An image obtained from the ultrasound emitting surface 28 corresponds to the fifth cross-section image 29.

The ultrasound probe 2 used in the present embodiments may be any of the ultrasound probes A, B, and C, and the present invention may be applied not only to the probe that is to be inserted into the body cavity of the test object, but also to an ultrasound probe for sending and receiving ultrasound waves between the body surface and the internal body, such as a probe used for an abdomen echo.

### <First embodiment>

The ultrasound diagnostic device 10 of the first embodiment is characterized in that it is provided with a reference image generating means (a reference image generator 12) for generating multiple reference images of arbitrary cross sections by using three-dimensional volume data of a test object, and a display means (a monitor 15) for displaying an ultrasound image and the reference images. The reference image generating means uses the cross-section position of the ultrasound image being calculated to generate at least one of the following; a first reference image made up of the same cross section as that of the ultrasound image, a second reference image made up of a cross section orthogonal to the ultrasound image and the ultrasound emitting surface, and a third reference image made up of a cross section being parallel to the ultrasound emitting surface. In other words, in the present embodiment, there are displayed the reference images of three cross sections being orthogonal to one another, and a guide image in which the cross-section positions of those reference images are superimposed on the three-dimensional test object image.

More specifically, in the first embodiment, the following reference images are generated from the three-dimensional volume data recorded in the volume data recorder 9; the first reference image made up of an image of the same cross section as that of the ultrasound image, the second reference image made up of an image of the cross section that is obtained by rotating the first reference image by 90° or by 270° around the depth direction of the ultrasound wave, and the third reference image made up of an image of the surface being parallel to the ultrasound emitting surface, and simultaneously, marks indicating the respective cross section positions are displayed in superimposed manner on the three-dimensional visible image of the test object. Further in the first embodiment, the operator uses a device such as a trackball on the ultrasound diagnostic device or a mouse being connectable to the ultrasound diagnostic device, not illustrated, thereby moving the marks indicating the various reference image positions on the guide image, and accordingly, the reference images are also updated and displayed. As described above, in the present embodiment, an explanation will be made taking an example that there are displayed reference images, one having the same cross section as that of the ultrasound image and biaxial cross sections orthogonal to the cross section of the ultrasound image. However, the cross sections of the reference images are not limited to those triaxial cross sections, but any cross sections obtained by moving and/or rotating those triaxial cross sections to arbitrary positions may be applicable.

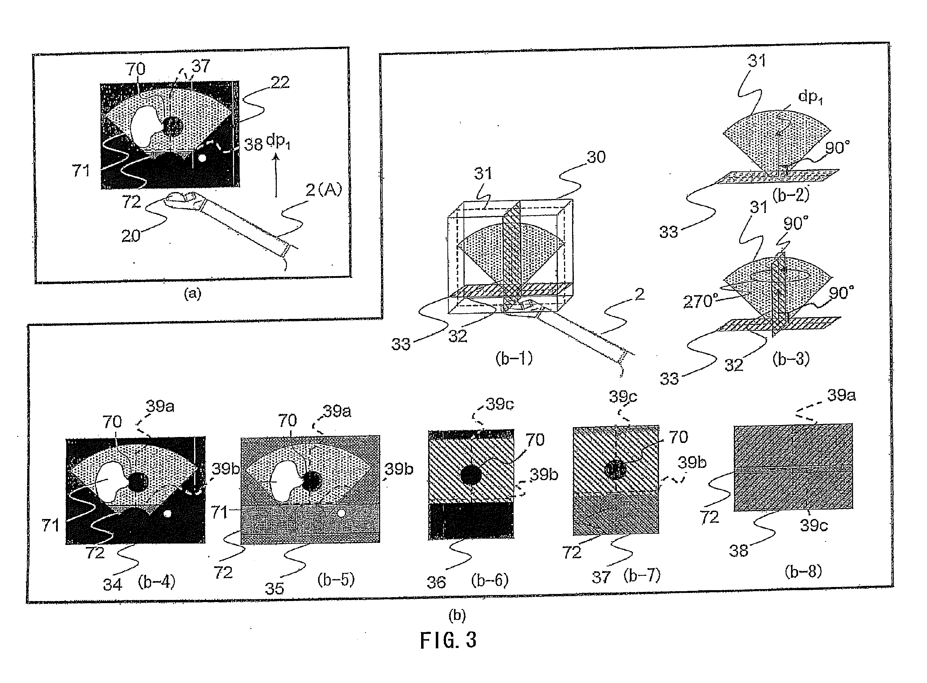

Firstly, an explanation will be made as to the positional relationship between the ultrasound emitting surface and the ultrasound image, and further the positional relationship between the ultrasound image and the reference images; the first reference image, the second reference image, and the third reference image. FIG. 3 illustrates the positional relationship between the ultrasound emitting surface and the ultrasound image, and also the positional relationship with the first reference image, the second reference image, and the third reference image; FIG. 3(a) illustrates a positional relationship between the ultrasound emitting surface and the ultrasound image emitted therefrom, and FIG. 3(b) illustrates a positional relationship of the cross sections that constitute the respective reference images. In the following explanation, the ultrasound probe A as shown in FIG. 2 is employed as the ultrasound probe 2, and the ultrasound image emitted from the ultrasound emitting surface 20 is taken as an example. The same explanation is applied to the case where the ultrasound emitting surface 21 of the ultrasound probe A is used.

As shown in FIG. 3(a), the ultrasound image 22 made up of the ultrasound waves emitted from the ultrasound emitting surface 20 corresponds to a region having a substantially fan-like shape expanding toward the depth direction dp1. Body organs 70, 71, and 72 in the test object are captured in the ultrasound image 22.

In the three-dimensional volume data 30 shown in (b-1) in FIG. 3(b), the cross section being the same as that of the ultrasound image 22 corresponds to the cross section 31, and the image of this cross section 31 is referred to as the first reference image in the present embodiment. When the cross section 31 being the first reference image is rotated by 90° or 270°, assuming the depth direction dp1 as the rotation axis, the cross section 32 is obtained (see (b-3) in FIG. 3(b)). An image of this cross section 32 is referred to as the second reference image in the present embodiment. The cross section 32 is orthogonal to the cross section 31. Therefore, the second reference image and the first reference image are orthogonal to each other.

In addition, an image of the cross section 33 that is parallel to the ultrasound emitting surface 20 is referred to as the third reference image in the present embodiment. The cross section 33 is orthogonal to the cross sections 31 and 32 (see (b-2) in FIG. 3(b)). Therefore, the third reference image is orthogonal to each of the first reference image and the second reference image.

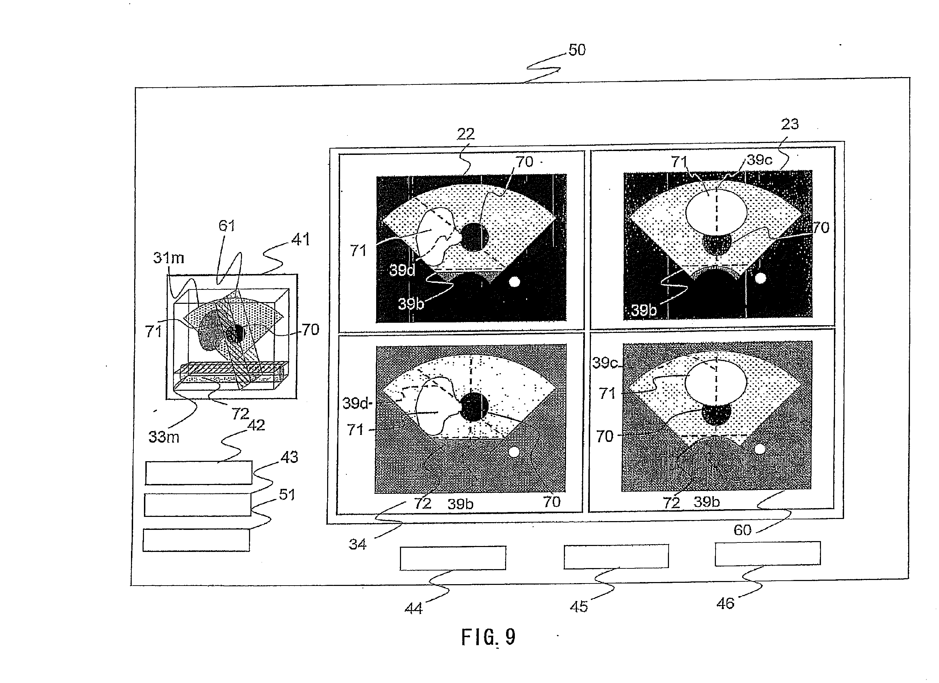

In the present embodiment, the ultrasound volume data is used as the three-dimensional volume data 30 to generate reference images that is surely provided with a real-time property. However, an MRI image or a CT image may constitute the three-dimensional volume data 30. It is to be noted that in the figures from FIG. 3 to FIG. 7, the ultrasound image 22, the FOV of the first reference image 31, and the mark 31m indicating the position of the first reference image 31 in the guide image, which will be described below, are depicted in dots. The cross section 32, the FOV of the second reference image 35, and the mark 32m indicating the position of the cross section 32 in the guide image, which will be described below, are depicted in hatching with falling diagonal strokes from top left to bottom right. And the cross section 33, the FOV of the third reference image 36, and the mark 33m indicating the position of the cross section 33 in the guide image, which will be described below, are depicted in hatching with falling diagonal strokes from top right to bottom left. In addition, the outside FOV is depicted using grid-like hatching with the following diagonal strokes; from top right to bottom left and from top left to bottom right. FIG. 9, which is referred to for explaining the second embodiment described below, employs the same hatching as the example described above.

The reference image generator 12 has a function for extracting the FOV and the outside FOV of the ultrasound image 22 obtained by the ultrasound probe A, and reducing the brightness of a region that corresponds to the outside FOV.

It is further possible to select displaying or hiding the reference image portion that corresponds to the outside FOV, according to the settings by the operator. Those functions above may bring clarity to the correspondence relationship between the ultrasound image and the reference image, thereby allowing the operator to easily grasp the correspondence relationship between both images.

The image 34 as shown in (b-4) in FIG. 3(b) and the image 35 as shown in (b-5) in the same figure are the first reference images that are generated by extracting the cross section 31 from the three-dimensional volume data 30. In the image 34, the region corresponding to the outside FOV is hidden, and in the image 35, the region corresponding to the outside FOV is displayed with its brightness level being lowered. The dotted line 39a within the images 34 and 35 indicates the position of the second reference image, and the dotted line 39b within the images 34 and 35 indicates the position of the third reference image. In other words, the reference image generator 12 displays on one reference image, a first mark indicating the cross section position of the other reference image in a superimposed manner thereon. This configuration facilitates grasping the positional relationship among the first reference image, the second reference image, and the third reference image being displayed.

Similarly, the image 36 shown in (b-6) in FIG. 3 (b), and the image 37 shown in (b-7) in the same figure correspond to the second reference images that are generated by extracting the cross section 32 from the three-dimensional volume data 30. In the image 36, the region corresponding to the outside FOV is hidden, and in the image 37, the region corresponding to the outside FOV is displayed with its brightness level being lowered. The dotted line 39c within the images 36 and 37 indicates the position of the first reference image, and the dotted line 39b within the images 36 and 37 indicates the position of the third reference image.

The image 38 shown in (b-8) in FIG. 3(b) corresponds to the third reference image that is generated by extracting the cross section 33 from the three-dimensional volume data 30, and the image 38 includes only the region that corresponds to the outside FOV. In the case of FIG. 3(b), the image is displayed with lowered brightness entirely. The dotted line 39a within the image 38 indicates the position of the second reference image, and the dotted line 39c indicates the position of the first reference image.

The aforementioned dotted lines 39a to 39c may be configured, for example, in such a manner that the color of the dotted line is changed for each cross section, or the dotted lines may be differentiated by display formats of various dotted line types, such as a dot-and-dash line, a chain double-dashed line, and a broken line, or both the color of the dotted line and the types thereof may be used to differentiate the dotted lines for the respective reference images. If the reference images are differentiated by the color of the dotted line, in order to clarify the correspondence relationship between the dotted line and the reference image, the display area of the reference image may be bordered with the color of the dotted line being associated, or an image identification mark with the same color or the same display format may be attached. It is the reference image generator 12 which executes the process for displaying the dotted lines 39a to 39c in such a manner as superimposed on the reference image.

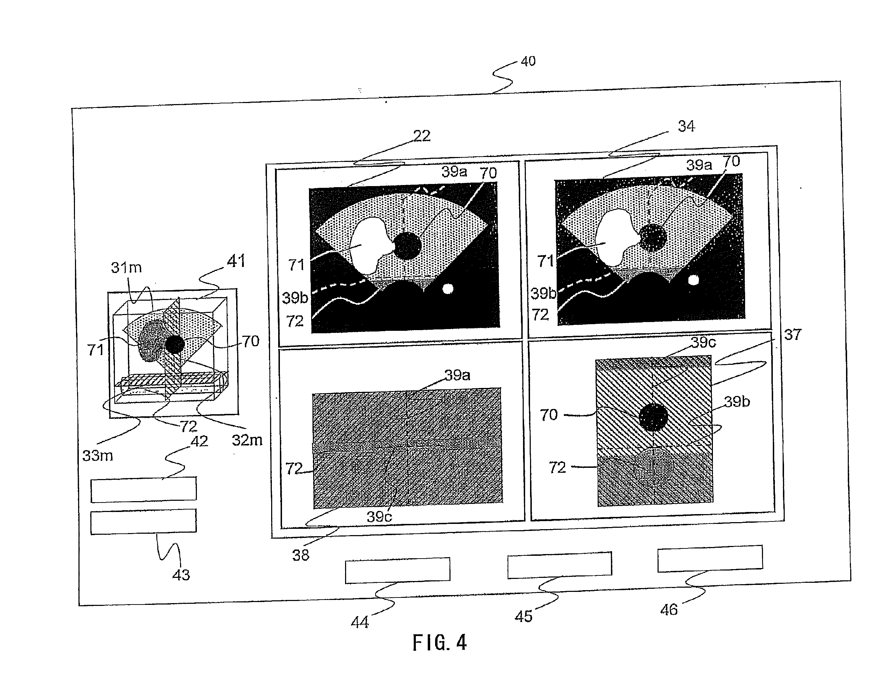

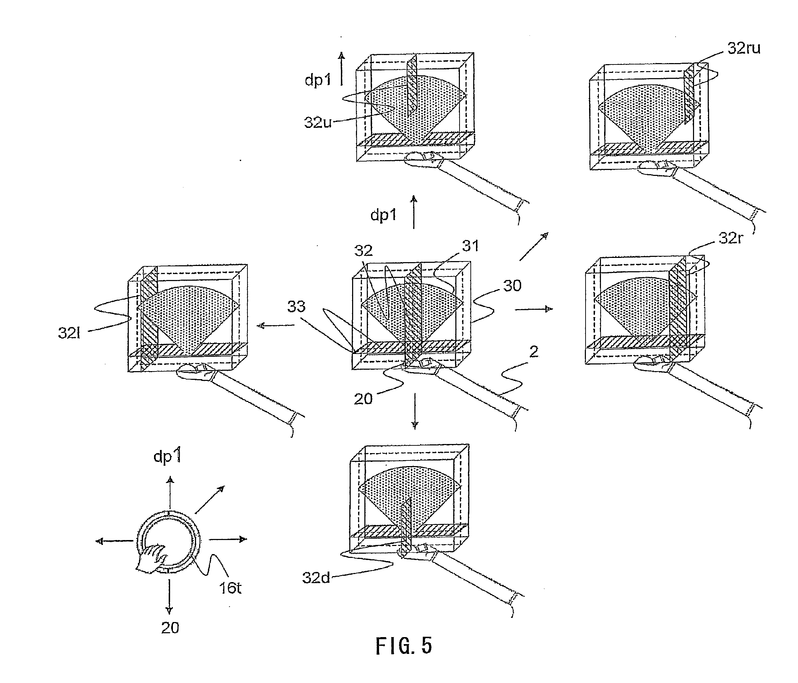

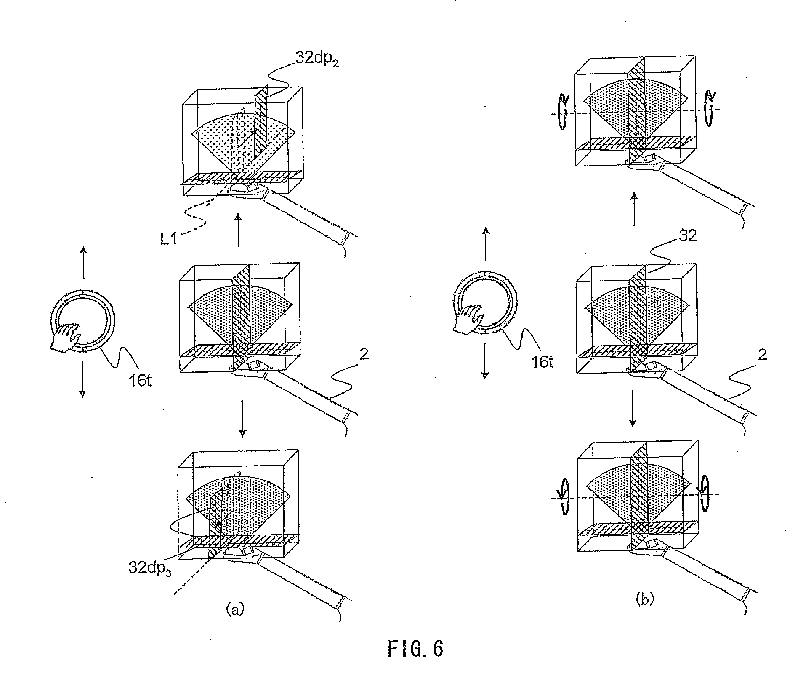

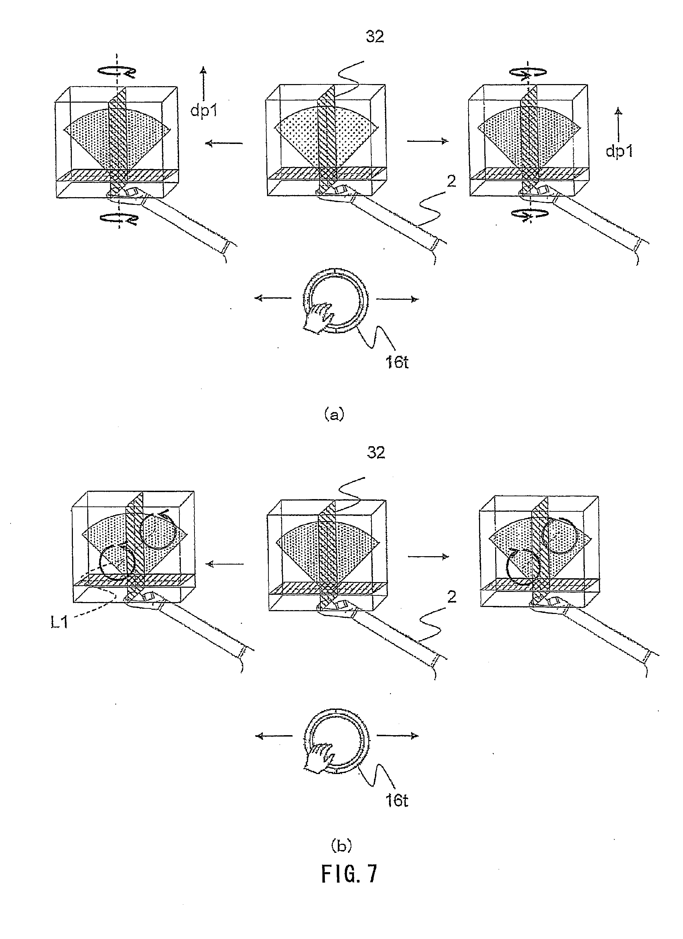

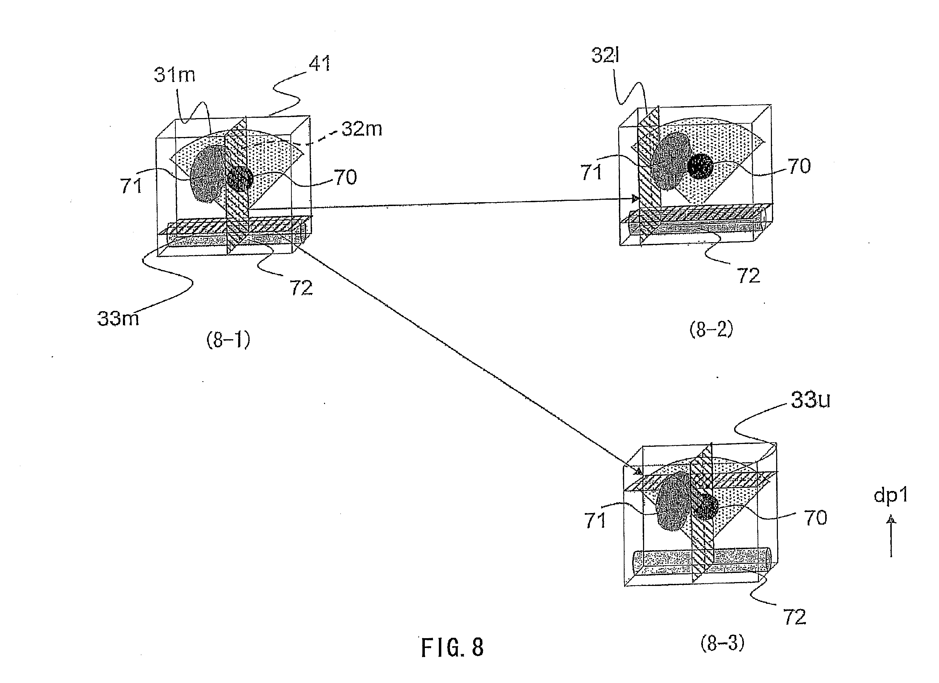

Next, with reference to the figures from FIG. 4 to FIG. 8, a display screen of the first embodiment will be explained. FIG. 4 illustrates a screen display example in the first embodiment. FIG. 5 illustrates a process for moving the second reference image, up and down, right and left, and in an oblique direction. FIG. 6 illustrates movement and rotation of the second reference image; FIG. 6(a) illustrates the state where the second reference image is moved along the front-and-rear direction, and FIG. 6(b) illustrates the state where the second reference image is rotated, assuming the direction for inserting the ultrasound prove as the rotation axis. FIG. 7 illustrates rotation of the second reference image; FIG. 7(a) illustrates the state where the second reference image is rotated assuming the depth direction as the rotation axis, and FIG. 7(b) illustrates the state where the second reference image is rotated assuming the front-and-rear direction as the rotation axis. FIG. 8 illustrates a method for moving the reference image according to the guide image.

As shown in FIG. 4, on the screen 40 relating to the present embodiment, there are displayed the ultrasound image 22, the first reference image 34 made up of the same cross section as that of the ultrasound image 22, the second reference image 37 obtained by rotating the first reference image 34 by 90° or by 270°, and the third reference image 38 being parallel to the ultrasound emitting surface, and the guide image 41. Furthermore, the screen 40 is provided with soft buttons allowing various functions to be executed. Those soft buttons include an ON/OFF switching button 42 for the guideline on the reference image, an ON/OFF switching button 43 for the guideline on the ultrasound image, a movement/rotation switching button 44, a moving direction (up and down, right and left, and oblique directions/rear and front directions) switching button 45, and the rotating direction (left and right, depth directions/rear and front directions) switching button 46 for the reference image.

The guide image 41 is the three-dimensionally visualized image, that visualizes the three-dimensional internal structure of the test object, obtained by applying volume rendering, for instance, based on the three-dimensional volume data of the test object imaged by an MRI device or a CT scanner. And the cross section 31 of the first reference image, the cross section 32 of the second reference image, and the cross section 33 of the third reference image, are displayed in a superimposing manner on the three-dimensionally visualized image.

When the operator presses the ON/OFF switching button 42 for the guideline on the reference image, it is possible to select displaying/hiding the dotted lines 39a, 39b, and 39c indicating the positions of other reference images being displayed in a superimposed manner, on the first reference image 34, the second reference image 37, and the third reference image 38.

When the operator presses the ON/OFF switching button 43 for the guideline on the ultrasound image, it is possible to select displaying/hiding the dotted lines 39a and 39b that are displayed also on the ultrasound screen. It is to be noted that the image processor 14 performs the processing for displaying the dotted lines 39a and 39b in a superimposed manner on the ultrasound image 22.

When the operator presses the movement/rotation switching button 44, it is possible to perform mode switching between moving and rotating, for the cross section 31 of the first reference image, the cross section 32 of the second reference image, and the cross section 33 of the third reference image.

When the moving direction switching button 45 is pressed, under the condition that "movement" is selected by the movement/rotation switching button 44 for the reference image, it is further possible to select either moving direction of the cross sections 31, 32, and 33 a combination of the up-and-down direction, the right-and-left direction and oblique directions, or the front-and rear direction. The "up-and-down direction" in the explanation above indicates the direction along the depth direction of the ultrasound wave, and the up direction corresponds to the direction along which the depth is increasing. The "right-and-left direction" corresponds to the direction being orthogonal to the depth direction of the ultrasound wave in the cross section of the ultrasound image. The "oblique direction" corresponds to any of the following directions in the cross section of the ultrasound image, the direction having an angle larger than 0° and smaller than 90°, the direction having an angle larger than 90° and smaller than 180°, the direction having an angle larger than 180° and smaller than 270°, and the direction having an angle larger than 270° and smaller than 360°, with respect to the depth direction of the ultrasound wave. In addition, the "font-and-rear direction" corresponds to the direction at right angle to both the depth direction of the ultrasound wave and the cross section of the ultrasound image.

When the rotating direction switching button 46 is pressed, under the condition that rotation is selected by the movement/rotation switching button 44 for the reference image, it is further possible to select the either rotating direction of the cross sections 31, 32, and 33 a combination of the rotation in the right-and-left direction and the rotation in the depth direction, or the rotation in the front-and-rear direction. The aforementioned "right-and-left direction" corresponds to a rotating direction assuming as the rotation axis, an axis that coincides with the depth direction on the same cross section as that of the ultrasound image. The "rotation in the depth direction" corresponds to the rotating direction assuming as the rotation axis, the axis that is orthogonal to the depth direction in the same cross section as that of the ultrasound image. The "rotation in the front-and-rear direction" corresponds to the rotating direction assuming as the rotating axis, an axis that coincides with the front-and-rear direction.

### (Movement in the up-and-down direction, the right-and-left direction, and the oblique direction)

With reference to FIG. 5, an explanation will be made as to a processing for moving the reference image in the up-and-down direction, the right-and-left direction, and the oblique direction. This processing corresponds to the case where the "up-and-down/right-and-left/oblique direction" is selected in the moving direction switching button 45 for the reference image. It is to be noted that in FIG. 5, the case where the second reference image is moved is taken as an example for the explanation, but the first reference image and the third reference image may be moved in the similar manner.

As shown in FIG. 5, the operator manipulates the track ball 16t, and the movement/rotation signal input part 16 calculates the moving direction and the moving amount of the image, according to the rotating direction and the rotating amount of the track ball 16t, and outputs the result to the reference image generator 12. The reference image generator 12 moves the cross section position of the second reference image or the third reference image, and generates and displays the second reference image or the third reference image after the movement.

In the three-dimensional volume data 30 as shown in FIG. 5, the cross section 31 of the first reference image, the cross section 32 of the second reference image, and the cross section 33 of the third reference image are displayed in such a manner as superimposed one on another. The positions of those cross sections 31, 32, and 33 on the three-dimensional volume data 30 are assumed as base positions.

When the operator rotates the track ball 16t upwardly, the cross section 32 moves in the upward direction with respect to the base position, in other words, in the depth direction dp1 of the ultrasound wave emitted from the emitting surface 20, and it is transferred to the cross section 32u. Similarly, when the operator rotates the track ball 16t in the upper-right direction, the right direction, the downward direction, and the left direction, the cross section 32 moves into the upper-right direction, the right direction, the downward direction, and the left direction, with respect to the base position, and the cross section 32 is transferred to the cross section 32ru, the cross section 32r, the cross section 32d, and the cross section 321. The aforementioned moving direction and the moving amount are just examples, and the cross section is movable in any direction by any amount.

### (Movement in the front-and-rear direction)

With reference to FIG. 6(a), an explanation will be made as to the movement along the front-and-rear direction. After the "movement" is selected according to the movement/rotation switching button 44 for the reference image, the "front-and-rear direction" is selected as the moving direction according to the moving direction switching button 45, and the operator rotates the track ball 16t upwardly. Then, the cross section 32 moves into the rear direction from the base position along the front-and-rear direction axis L1, and it is transferred to the cross section 32dp₂. On the other hand, when the operator rotates the track ball 16t downwardly, the cross section 32 moves from the base position toward the front, and it is transferred to the cross section 32dp₃.

### (Rotating movement)

With reference to FIG. 6(b) and FIG. 7, the rotation of the reference image will be explained.

In the case where the rotating direction is controlled, after the "rotation" is selected according to the movement/rotation switching button 44 and the "right-and-left/ depth direction" is selected as the rotating direction according to the rotation direction switching button 46, the operator rotates the track ball 16t in the up-and-down direction. Then, the second reference image rotates assuming as the rotation axis, the right-and-left direction of the first reference image (FIG. 6(b)). Similarly, when the track ball 16t is rotated in the right-and-left direction, the second reference image rotates assuming as the rotation axis, the depth direction dp1 (FIG. 7(a)).

In addition, after selecting the "front-and-rear direction" as the rotating direction according to the rotating direction switching button 46, and the track ball 16t is rotated in the right-and-left direction. Then, the second reference image is rotated assuming the front-and-rear direction axis L1 as the rotation axis (FIG. 7(b)).

### (Movement of the reference image according to the guide image)

As shown in FIG. 8, the guide image generator 13 displays the cross sections 31m, 32m, and 33m respectively indicating the positions of the first, the second, and the third reference images on the guide image 41. FIG. 8 (8-1) shows the cross sections 31m, 32m, and 33m at the base positions. In this situation, when the operator selects the cross section 32m of the second reference image by the mouse and drags it in the left direction as shown in FIG. 8 (8-2), it is transferred to the cross section 321, and the second reference image cut out from the cross section 321 is generated. In addition, when the operator selects the cross section 33m of the third reference image by the mouse and drags it in the depth direction dp1 of the ultrasound wave, as shown in FIG. 8 (8-3), the cross section 33m is transferred to the cross section 33u, and the third reference image cut out from the cross section 33u is generated.

In the present embodiment, after any of the first reference image, the second reference image, and the third reference image is designated by the pointing device, it is subjected to the moving and rotating control as described above, and only the designated reference image is moved and rotated. In addition, following the positioning and rotation of the second reference image and the third reference image, the guide image generator 13 moves or rotates also the cross sections 32m and 33m indicating the positions of the second reference image and the third reference image on the guide image 41, in the up-and-down direction, the right-and-left direction, the oblique direction, and the front-and-rear direction, and displays those cross sections.

It is to be noted that colors used for the color coding of the cross sections 31m, 32m, and 33m are displayed in the same colors as those of the dotted lines 39a, 39b, and 39c indicating the positions of the first, second, and third reference images, being associated respectively. Accordingly, this allows the correspondence relationship to be clarified more, between the marks 31m, 32m, and 33m indicating the positions of the reference images on the guide image 41, and the respective reference images.

According to the present embodiment, there are provided the ultrasound probe 2 having the ultrasound emitting surface for emitting an ultrasound wave and receiving a reflected wave of the ultrasound wave, an ultrasound image generating means (an ultrasound image generator 6) for generating the ultrasound image using a reflected echo signal based on the reflected wave, a reference image generating means (a reference image generator 12) for generating reference images of multiple arbitrary cross sections, by using the three-dimensional volume data of the test object, and a display means (a monitor 15) for displaying the ultrasound image and the reference images. In addition, the ultrasound image display method is provided, including a step of emitting an ultrasound wave and generating an ultrasound image by using a reflected echo signal based on a reflected wave of the ultrasound wave, a step of generating reference images of multiple arbitrary cross sections by using the three-dimensional volume data of the test object, and a step of displaying the ultrasound image and the reference images.

The reference image generating means (the reference image generator 12) displays one reference image with the first mark indicating the cross section position of the other reference image in such a manner as superimposed thereon. The first mark that is superimposed on one reference image, and the mark of the other reference image obtained from the cross section position indicated by the first mark are displayed using the same display format. There are further provided the positional information detecting means (the position sensor 4) for detecting the positional information of the ultrasound probe 2, and the scan plane acquiring means (the scan plane acquisition part 11) for calculating the cross section position of the ultrasound image, based on the positional information, and the reference image generating means (the reference image generator 12) uses the cross section position of the ultrasound image being calculated, to generate at least one of the following; the first reference image made up of the same cross section as that of the ultrasound image, the second reference image orthogonal to the ultrasound image and the ultrasound emitting surface, and the third reference image made up of the cross section being parallel to the ultrasound emitting surface.

There is further provided the first input means (the movement/rotation signal input part 16) for accepting at least either of the moving direction and moving amount, and the rotating direction and rotating amount of the reference image, and the reference image generating means (the reference image generator 12) generates, according to the input value accepted by the first input means (the movement/rotation signal input part 16), a reference image made up of the cross section that is obtained by moving and rotating an arbitrary cross section. Then, the display means (the monitor 15) updates and displays the reference image being generated. There is further provided the image processing means (the image processor 14) for displaying on the ultrasound image, a second mark indicating the cross section position of the reference image in such a manner as superimposed thereon. The second mark that is superimposed on the ultrasound image, and the mark of the reference image obtained from the cross section position indicated by the second mark are displayed using the same display format.

There is further provided the guide image generating means (the guide image generator 13) for generating a guide image on which a third mark indicating the cross section position of the reference image is displayed in such a manner as superimposed on the three-dimensional image of the test object. There is further provided the second input part for accepting an input of at least either of the moving direction and the moving amount, and the rotating direction and the rotating amount of the third mark that is superimposed on the guide image. And the reference image generating means (the reference image generator 12) generates a reference image based on the cross section being associated with the third mark that is moved and rotated according to the input value accepted by the second input means. Then, the display means (the monitor 15) updates and displays the reference image being generated.

The third mark that is superimposed on the guide image and the mark of the reference image obtained from the cross section position indicated by the third mark are displayed using the same display format.

There is further provided an operating means for changing the FOV of the ultrasound wave emitted from the ultrasound emitting surface. The ultrasound image generating means (the ultrasound image generator 6) newly generates an ultrasound image being associated with the FOV after the change. The reference image generating means (the reference image generator 12) newly generates reference images associated with the magnification of the newly generated ultrasound image. And the display means (the monitor 15) updates and displays the ultrasound image and the reference images newly generated.

Accordingly, the second reference image and the third reference image are allowed to move and rotate freely, in the depth direction, the right-and-left direction, the oblique direction, and the front-and-rear direction. Therefore, it is possible to grasp a structure of the body cavity forward of the ultrasound probe, and also a structure of the body cavity such as a portion that is difficult to observe by the ultrasound probe, without inserting the ultrasound probe into the body cavity deeply. In addition, the guide image 41 indicating the positions of the first, the second, and the third reference images is simultaneously displayed, thereby facilitating to grasp the positional relationship among the first, the second, and the third reference images, and accordingly, this may mitigate the burden on the test object and the operator. With the technique as described above, the present invention may also be used as a navigation tool. In addition, the color of the mark (dotted line 39a, etc.,) that is superimposed on the reference image, the guide image, and the ultrasound image may be made identical to the border color of the each reference image or the color of the image identification mark, or the same display format may be employed, thereby facilitating to grasp the cross section position of the reference image, and consequently, it becomes easy to know which portion of the test object is observed upon performing a diagnosis.

### <Second embodiment>

In the second embodiment, a biplane ultrasound probe (the ultrasound probe A as shown in FIG. 2) is employed as the ultrasound probe, and there are displayed two ultrasound images obtained from the ultrasound probe A in real time, and reference images which are extracted from the cross sections being identical to those of the ultrasound images, as the reference images being associated with the ultrasound images, respectively.

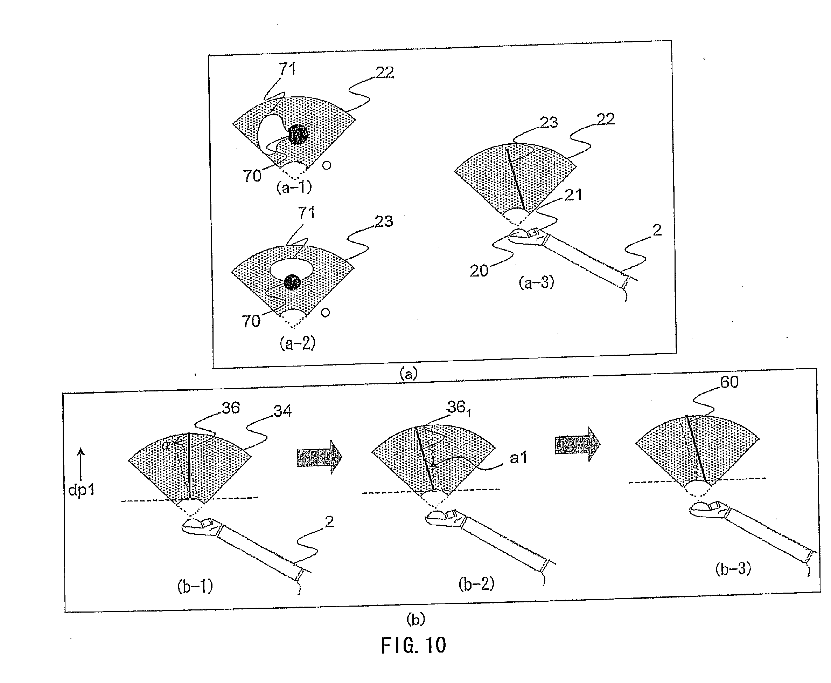

Hereinafter, with reference to the figures from FIG. 9 to FIG. 11, the second embodiment will be explained. FIG. 9 is a schematic diagram illustrating a screen display example being displayed in the second embodiment. FIG. 10 illustrates a process for generating the reference images that are displayed in the second embodiment; FIG. 10 (a) illustrates the tip of the ultrasound probe A, and FIG. 10(b) illustrates a process for generating the reference image 60 from the second reference image 36.

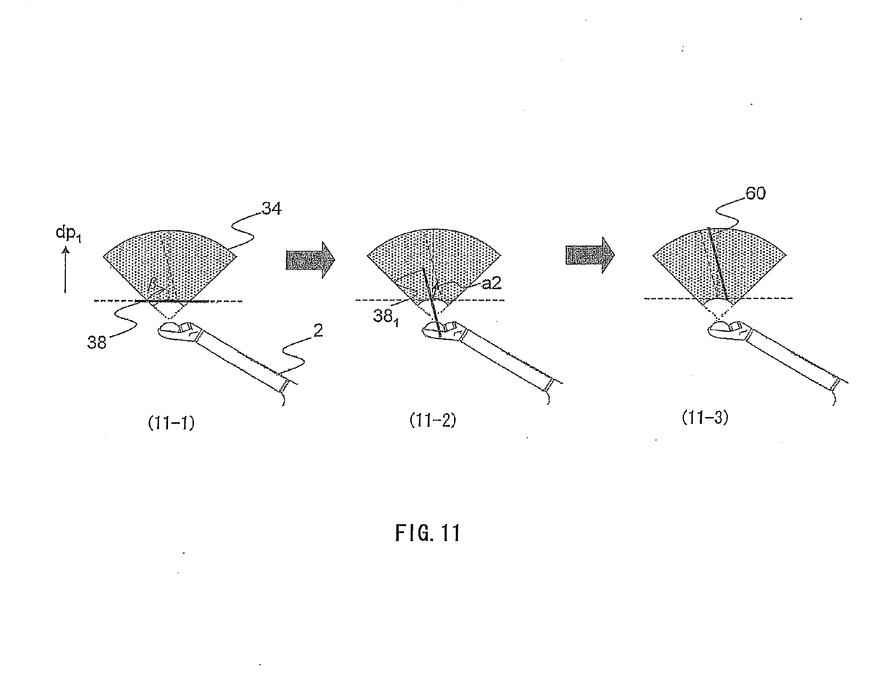

FIG. 11 illustrates a process for generating the reference image 60 from the third reference image 38.

In the screen 50 as shown in FIG. 9, an image selection button 51 is added to the screen layout that is shown in the display screen layout relating to the first embodiment (see FIG. 4). When this image selection button 51 is pressed, an image that is desired to be moved and rotated is selected, and when the track ball or a mouse wheel of the mouse is turned, this allows only the selected image to be moved and rotated. An image being selectable may be not only the image being displayed, but also the image being hidden. By way of example, when the third reference image being hidden is selected by the image selection button 51, and then the track ball or the mouse wheel of the mouse is turned, this moves the position of the third reference image being hidden in FIG. 9, and following this movement, the dotted line 39b indicating the position of the third reference image and the mark 33m indicating the position of the third reference image on the guide image 41 are allowed to move.

On the screen 50, in the display area of the ultrasound image, there is displayed on the upper left, the first cross-section image 22 (hereinafter, referred to as the "ultrasound image 22") obtained from the ultrasound emitting surface 20, and there is displayed on the upper right, the second cross-section image 23 (hereinafter, referred to as the "ultrasound image 23") obtained from the ultrasound emitting surface 21. On the lower left, there is displayed the first reference image 34 that is reconstructed on the cross section 31 based on the volume data 30, as the reference image of the ultrasound image 22. On the lower right, there is displayed the reference image 60 of the ultrasound image 23. The reference image 60 is an image, after moving and rotating the second reference image 36 (see FIG. 10(b)) of the ultrasound image 22 obtained from the ultrasound emitting surface 20, or moving and rotating the third reference image 38 (see FIG. 11), having the same cross section and being expanded and reduced at the same magnification as those of the ultrasound image 23. In other words, the reference image 60 of the ultrasound image 23 is generated on the basis of the second reference image 36 or the third reference image 38 of the ultrasound image 22. It is of course possible to reconstruct the reference image 60 of the ultrasound image 23 based on the volume data 30, and display the image as the first reference image of the ultrasound emitting surface 21, or it is further possible to move and rotate the second reference image or the third reference image of the ultrasound image 23 obtained from the ultrasound emitting surface 21, and generate an image having the same cross section and being expanded and reduced at the same magnification, as those of the ultrasound image 23.

With reference to FIG. 10 and FIG. 11, an explanation will be made as to the process for generating the reference image 60 of the ultrasound image 23, based on the second reference image 36 or the third reference image 38 of the ultrasound image 22.

As shown in (a-3) in FIG. 10(a), the tip of the ultrasound probe A is provided with the ultrasound emitting surfaces 20 and 21. From the ultrasound emitting surface 20, the ultrasound image 22 is obtained (see FIG. 10 (a) (a-1)). From the ultrasound emitting surface 21, the ultrasound image 23 is obtained (see FIG. 10(a) (a-2)). The organ 70 and organ 71 are imaged in each of the ultrasound images 22 and 23.

The reference image generator 12 acquires from the ultrasound probe 2 in advance, a deviation angle α° between the depth direction axis dp1 of the ultrasound emitting surface 20, and the depth direction axis of the ultrasound emitting surface 21. Alternatively, the coordinates of ultrasound emitting surface 20 and the ultrasound emitting surface 21 are acquired from the scan plane acquisition part 11, and the deviation angle α° in the depth direction may be calculated by using those coordinates.

As shown in (b-1) in FIG. 10(b), the reference image generator 12 generates the first reference image 34 and the second reference image 36 of the ultrasound image 23. As described above, this first reference image 34 is displayed just below the ultrasound image 22 on the screen 50, as the reference image of the ultrasound image 23.

The reference image generator 12 rotates the second reference image 36 by α°. With this rotation, the second reference image 36 makes a transit to the image 36₁ (see FIG. 10(b) (b-2)). Next, the reference image generator 12 uses the coordinates of the image 36₁ and the coordinates of the ultrasound image 23, to move the image 36₁ and adjust the size and the FOV thereof in order to make coincidence therebetween (FIG. 10(b)(b-2) illustrates the moving amount and direction by the arrow a1). Accordingly, on the basis of the second reference image 36, the reference image 60 having the same cross section as that of the ultrasound image 23 is generated (see FIG. 10(b) (b-3)).

Alternatively, as for those reference images, angle β° is acquired in advance from the ultrasound probe 2, the angle allowing the inclination of the third reference image 38 of the ultrasound image 22 to coincide with the inclination of the reference image 60 having the same cross section as that of the ultrasound image 23, according to the positional relationship between the ultrasound emitting surface 20 and the ultrasound emitting surface 21. Further alternatively, the coordinates of the ultrasound emitting surface 20 and the ultrasound emitting surface 21 are acquired from the scan plane acquisition part 11, the deviation angle β° is calculated by using those coordinates. Then, the third reference image 38 is rotated by β° (see FIG. 11 (11-1)). With this rotation, the third reference image 38 makes a transit to the image 38₁ (see FIG. 11 (11-2)). Next, the reference image generator 12 uses the coordinates of the image 38₁ and the coordinates of the ultrasound image 23, to move the image 38₁ and adjust the size and the FOV thereof in order to make coincidence therebetween (FIG. 11 (11-2) illustrates the moving amount and direction by the arrow a2). Accordingly, on the basis of the third reference image 38, the reference image 60 having the same cross section as that of the ultrasound image 23 is generated (see FIG. 11 (11-3)).

The reference image that is not used for generating the reference image 60 is hidden; that is, the third reference image 38 when the reference image 60 is generated by using the second reference image 36, or the second reference image 36 when the reference image 60 is generated by using the third reference image 38 is hidden. However, the dotted line indicating the position of the reference image being hidden (in FIG. 9, the dotted line 39b indicating the position of the third reference image) and the dotted line 39d indicating the position of the reference image 60 are displayed in a superimposed manner on the ultrasound image 22 and the first reference image 34. Similarly, on the ultrasound image 23 and the reference image 60, the dotted line 39c indicating the position of the first reference image being displayed, and the dotted line of the reference image being hidden (in FIG. 9, the third reference image position 39b) are displayed in such a manner as superimposed thereon.

The colors of the dotted lines 39d and 39c are respectively the same as the colors of the borders of the reference image 60 and the first reference image 34, in order to clarify the association between the reference image 60 and the first reference image 34, and according to the setting by the operator, it is possible to select displaying or hiding those dotted lines.

In addition, on the guide image 41, the display indicating the position of the second or the third reference image that was used for generating the reference image 60 is set to be hidden automatically, and instead, the mark 61 indicating the position of reference image 60 is displayed. The mark 61 indicating the position of the reference image 60 has the same color as the color of the dotted line 39d indicating the position of the reference image 60 and the border color of the reference image 60, in order to clarify the association with the reference image 60.

It is to be noted that the ultrasound images 22 and 23 obtained from the ultrasound emitting surfaces 20 and 21, the first reference image 34, the reference image 60, and the guide image 41, are exchanged therebetween, by dragging and dropping via the track ball and the keyboard on the ultrasound device not illustrated, or by dragging and dropping via the mouse not illustrated, being connectable with the device.

According to the present embodiment, the ultrasound probe 2 is provided with multiple ultrasound emitting surfaces, for simultaneously acquiring ultrasound images of different cross sections from the respective ultrasound emitting surfaces. The reference image generating means (the reference image generator 12) generates the reference images being associated with the respective ultrasound images of the different cross sections. And the display means (the monitor) 15 simultaneously displays the multiple ultrasound images and multiple reference images being associated with the respective ultrasound images.

There are displayed two ultrasound images 22 and 23, and their reference images that are adjusted in such a manner that the position, angle, magnification, FOV, and the like, become identical to those of the respective ultrasound images 22 and 23, and the guide image 41, whereby the operator is allowed to observe an affected area while referring to the ultrasound images and the reference images, and grasp the inside of the body cavity more precisely than the conventional technique, and this facilitates an examination and a diagnosis. In addition, since the guide image 41 is generated from three-dimensional volume data, it is possible to provide the operator with beneficial information such as information of a portion to be observed, for instance, that is obtained from an angle different from the angle of an ultrasound tomographic image, thereby enhancing diagnostic performance, eventually.

### <Third embodiment>

In the third embodiment, when a non-biplane ultrasound probe (the ultrasound probe B in FIG. 2) is employed as the ultrasound probe, an ultrasound image in real time, and the second and third reference images for the ultrasound image are combined to perform pseudo-biplane display via the reference images.

It is assumed, for instance, that with the ultrasound probe B (see FIG. 2), a third cross-section image 26 (hereinafter, referred to as an "ultrasound image 26") is obtained from the ultrasound emitting surface 24, and a fourth cross-section image 27 (hereinafter, referred to as an "ultrasound image 27") is obtained from the ultrasound emitting surface 25. Then, this ultrasound probe is allowed to perform switching between the ultrasound emitting surfaces 24 and 25, according to the ultrasound emitting surface switching button (not illustrated) on the ultrasound diagnostic device 10 or on the monitor 15. In the present embodiment, reference images respectively associated with the ultrasound images 26 and 27 are generated for the ultrasound probe, from the second reference image or the third reference image (or the first reference image).

By way of example, when the operator selects the ultrasound emitting surface 24 of the ultrasound probe B, the ultrasound image 26 from the ultrasound emitting surface 24 is displayed. Here, it is not possible for the non-biplane ultrasound probe to obtain the ultrasound image 27 from the ultrasound emitting surface 25, simultaneously. Therefore, the reference image generator 12 uses the second reference image or the third reference image (or the first reference image) for the ultrasound image 26 to generate a reference image of the same cross section as that of the ultrasound image 27, and display the reference image thus generated. Accordingly, there are displayed according to the non-biplane ultrasound probe, the ultrasound image obtained from the selected ultrasound emitting surface, and the reference image obtained from the same cross section as that of the ultrasound image obtained from the ultrasound emitting surface that is not selected. It is possible to implement the processing for generating an image of the same cross section as that of the ultrasound image 27, with the use of the second reference image or the third reference image (or the first reference image) for the ultrasound image 26, by replacing the ultrasound image 23 in the second embodiment, with the ultrasound image 26.

As opposite to the example above, if the ultrasound emitting surface 25 is selected, the ultrasound image 27 and a reference image having the same cross section as that of the ultrasound image 26 are displayed, the reference image is generated by using the second reference image or the third reference image for the ultrasound image 27. It is to be noted that in the aforementioned above, the ultrasound image 26, and the reference image having the same cross section as that of the ultrasound image 27 are displayed, but reference images associated with the respective images may also be displayed.

According to the third embodiment, the ultrasound probe 2 is provided with multiple ultrasound emitting surfaces, this ultrasound probe being to acquire an ultrasound image from either one of the ultrasound emitting surfaces being selected. The reference image generating means (the reference image generator 12) generates a reference image having the same cross section as that of the ultrasound image not selected. And the display means (the monitor 15) simultaneously displays the ultrasound image of any selected one ultrasound emitting surface, and the reference image having the same cross section as that of the ultrasound image not selected. When the non-biplane ultrasound probe is used, an ultrasound image supposed to be obtained from the emitting surface that is not selected, is generated and displayed, based on the first, the second, or the third reference image for the ultrasound image that is obtained from the emitting surface being selected, thereby implementing a display mode that is similar to the case where a biplane ultrasound probe is employed.

### <Fourth embodiment>

In the fourth embodiment, the cross section positions of the first reference image, the second reference image, and the third reference image, being orthogonal to one another are set in such a manner that a desired area observed by the operator is included therein.

Similar to the ultrasound diagnostic device 10 of the first embodiment, the ultrasound diagnostic device 10 of the fourth embodiment is provided with a reference image generating means (the reference image generator 12) for generating reference images of multiple arbitrary cross sections by using three-dimensional volume data of a test object, and a display means (the monitor 15) for displaying the ultrasound image and the reference images. The reference image generating means (the reference image generator 12) uses the cross section position of the ultrasound image being calculated, to generate the first reference image made up of the same cross section as that of the ultrasound image, the second reference image made up of the cross section being orthogonal to the ultrasound image and the ultrasound emitting surface, or the third reference image made up of the cross section being parallel to the ultrasound emitting surface.

Here, the reference image generating means (the reference image generator 12) generates the first reference image of the cross section being the same as that of the ultrasound image, and adjusts the cross section positions of the second reference image and the third reference image based on an amount of characteristics of the ultrasound image, and generates the second reference image and the third reference image made up of the cross sections that are orthogonal to the first reference image. The amount of characteristics of the ultrasound image includes brightness information, values of elasticity (distortion, elastic modulus, etc.), blood flow information, and the like.

In the case where the ultrasound image includes a low-intensity region, the reference image generating means (the reference image generator 12) generates the first reference image having the same cross section as that of the ultrasound image, adjusts the cross section positions of the second reference image and the third reference image in such a manner that the second reference image and the third reference image include the low-intensity region (e.g., a portion having the lowest intensity), and generates the second reference image and the third reference image.

As shown in FIG. 3(a), in the ultrasound image 22 made up of the ultrasound wave emitted from the emitting surface 20, there is displayed the organ 70 within the test object. In the present embodiment, if it is assumed that the organ 70 displayed in the ultrasound image 22 corresponds to the low-intensity region, the reference image generating means (the reference image generator 12) adjusts the cross section positions of the second reference image and the third reference image in such a manner that the second reference image and the third reference image include the organ 70, and generates the second reference image and the third reference image.

In addition, if the ultrasound image includes a robust region (a robust region based on the value of elasticity), the reference image generating means (the reference image generator 12) generates the first reference image having the same cross section as that of the ultrasound image, adjusts the cross section positions of the second reference image and the third reference image in such a manner that the second reference image and the third reference image include the robust region (e.g., a portion having small distortion or a low value of elasticity), and generates the second reference image and the third reference image. The value of elasticity is a value that is calculated by detecting displacement between multiple ultrasound images, for instance. As the value of elasticity, it is possible to calculate from the displacement, distortion and an elasticity coefficient.

As shown in FIG. 3(a), in the ultrasound image 22 made up of the ultrasound wave emitted from the ultrasound emitting surface 20, there is displayed the organ 70 within the test object. In the present embodiment, if it is assumed that the organ 70 displayed in the ultrasound image 22 corresponds to a robust region, the reference image generating means (the reference image generator 12) adjusts the cross section positions of the second reference image and the third reference image in such a manner that the second reference image and the third reference image include the organ 70, and generates the second reference image and the third reference image.

If the ultrasound image includes blood flow information (Doppler information), the reference image generating means (the reference image generator 12) generates the first reference image having the same cross section as that of the ultrasound image, adjusts the cross section positions of the second reference image and the third reference image in such a manner that the second reference image and the third reference image include the blood flow information, and generates the second reference image and the third reference image.

As shown in FIG. 3(a), in the ultrasound image 22 made up of the ultrasound wave emitted from the ultrasound emitting surface 20, there is displayed the organ 70 within the test object. In the present embodiment, if it is assumed that there is a blood flow in the organ 70 that is displayed in the ultrasound image 22, the reference image generating means (the reference image generator 12) adjusts the cross section positions of the second reference image and the third reference image in such a manner that the second reference image and the third reference image include the organ 70, and generates the second reference image and the third reference image.

According to the fourth embodiment, it is possible to display the first reference image, the second reference image, and the third reference image being orthogonal to one another, so that a desired area observed by the operator is included therein.

### Explanation of References

1 ultrasound diagnostic device main body, 2 ultrasound probe, 3 position sensor fixing mechanism, 4 position sensor, 5 source origin, 6 ultrasound image generator, 7 memory, 8 ultrasound volume data generator, 9 volume data recorder, 10 ultrasound diagnostic device, 11 scan plane acquisition part, 12 reference image generator, 13 guide image generator, 14 image processor, 15 monitor, 16 movement/rotation amount input part, 17 medical diagnostic imaging device

## Claims

1. An ultrasound diagnostic device comprising,
an ultrasound probe having an ultrasound emitting surface which emits an ultrasound wave and receives a reflected wave of the ultrasound wave,
an ultrasound image generating means for generating an ultrasound image by using an reflected echo signal based on the reflected wave,
a reference image generating means for generating reference images of multiple arbitrary cross sections by using a three-dimensional volume data of a test object, and
a display means for displaying the ultrasound image and the reference images.

2. The ultrasound diagnostic device according to claim 1, wherein,
the reference image generating means displays a first mark in a superimposed manner on one reference image, the first mark indicates a cross section position of another reference image.

3. The ultrasound diagnostic device according to claim 2, wherein,
the first mark that is superimposed on the one reference image, and a mark indicating the another reference image obtained from the cross section position indicated by the first mark, are displayed using an identical display format.

4. The ultrasound diagnostic device according to claim 1, further comprising,
a positional information detecting means for detecting positional information of the ultrasound probe, and
a scan plane acquiring means for calculating a cross section position of the ultrasound image, based on the positional information, wherein,
the reference image generating means uses the cross section position of the ultrasound image being calculated, to generate at least one of the following; a first reference image made up of the same cross section as that of the ultrasound image, a second reference image orthogonal to the ultrasound image and the ultrasound emitting surface, and a third reference image made up of the cross section being parallel to the ultrasound emitting surface.

5. The ultrasound diagnostic device according to claim 1, further comprising,
a first input means for accepting at least either of inputs; a moving direction and a moving amount, and a rotating direction and a rotating amount of the reference image, wherein,
the reference image generating means generates, according to values of the inputs accepted by the first input means, a reference image made up of the cross section that is obtained by moving and rotating the arbitrary cross section, and
the display means updates and displays the reference image being generated.

6. The ultrasound diagnostic device according to claim 1, further comprising,
an image processing means for displaying a second mark indicating a cross section position of the reference image in a superimposed manner on the ultrasound image.

7. The ultrasound diagnostic device according to claim 6, wherein,
the second mark that is superimposed on the ultrasound image, and a mark of the reference image obtained from the cross section position indicated by the second mark are displayed using an identical display format.

8. The ultrasound diagnostic device according to claim 1, further comprising,
a guide image generating means for generating a guide image on which a third mark indicating a cross section position of the reference image is displayed in such a manner as superimposed on a three-dimensional image of the test object.

9. The ultrasound diagnostic device according to claim 8, further comprising,
a second input means for accepting inputs of at least either of a moving direction and a moving amount, and a rotating direction and a rotating amount of the third mark that is superimposed on the guide image, wherein,
the reference image generating means generates a reference image based on the cross section being associated with the third mark that is moved and rotated according to values of the inputs accepted by the second input means,
the display means updates and displays the reference image being generated.

10. The ultrasound diagnostic device according to claim 8, wherein,
the third mark that is superimposed on the guide image, and a mark of the reference image obtained from the cross section position indicated by the third mark are displayed using an identical display format.

11. The ultrasound diagnostic device according to claim 1, further comprising,
an operating means for changing an FOV of the ultrasound wave emitted from the ultrasound emitting surface, wherein,
the ultrasound image generating means newly generates an ultrasound image being associated with the FOV after the change,
the reference image generating means newly generates a reference image associated with the magnification of the newly generated ultrasound image, and
the display means updates and displays the ultrasound image and the reference images newly generated.

12. The ultrasound diagnostic device according to claim 1, wherein,
the ultrasound probe comprises multiple ultrasound emitting surfaces for simultaneously acquiring ultrasound images of different cross sections, respectively from the ultrasound emitting surfaces,
the reference image generating means generates the reference images respectively associated with the ultrasound images of the different cross sections,
the display means simultaneously displays the multiple ultrasound images, and multiple reference images respectively associated with the ultrasound images.

13. The ultrasound diagnostic device according to claim 1, wherein,
the ultrasound probe comprises multiple ultrasound emitting surfaces for acquiring an ultrasound image from either one of the ultrasound emitting surfaces being selected,
the reference image generating means generates a reference image having the same cross section as that of the ultrasound image not selected, and
the display means simultaneously displays the ultrasound image obtained from any selected one ultrasound emitting surface, and the reference image having the same cross section as that of the ultrasound image not selected.

14. The ultrasound diagnostic device according to claim 1, wherein,
the reference image generating means generates a first reference image of the cross section being the same as that of the ultrasound image, adjusts cross section positions of a second reference image and a third reference image made up of cross sections that are orthogonal to the first reference image, based on an amount of characteristics in the ultrasound image, and generates the second reference image and the third reference image.

15. An ultrasound image display method comprising,
a step of emitting an ultrasound wave and generating an ultrasound image by using a reflected echo signal based on a reflected wave of the ultrasound wave,
a step of generating reference images of multiple arbitrary cross sections by using a three-dimensional volume data of a test object, and
a step of displaying the ultrasound image and the reference images.
